# EUROPÄISCHE PATENTSCHRIFT

(11) **EP 0 622 626 B1**
(45) Veröffentlichungstag und Bekanntmachung des Hinweises auf die Patenterteilung: **06.03.2002**
(21) Anmeldenummer: 94105883.6
(22) Anmeldetag: 15.04.1994
(51) Int. Cl.: G01N 21/86, B65D 83/04, B65D 75/70, B65D 75/58

(54) **System zur Analyse von Inhaltsstoffen flüssiger Proben**
System for analysing the components of fluid samples
Système d'analyse des composants d'échantillons fluides

(30) Priorität: 23.04.1993 DE 4313252; 27.08.1993 DE 4328816
(43) Veröffentlichungstag der Anmeldung: 02.11.1994
(60) Teilanmeldung: 01113369.1
(73) Patentinhaber: Roche Diagnostics GmbH, 68298 Mannheim (DE)
(72) Erfinder: Schreiber, Jörg,Dr., D-68542 Heddesheim (DE); Schmid, Wilfried,Dr., D-68259 Mannheim (DE); Kuhr, Hans-Jürgen,Dr., D-68219 Mannheim (DE); Eikmeier, Heino Dr.,c/o Boehringer Mannheim Corp., Indianapolis, Indiana 46250 (US); Sacherer, Klaus-Dieter, D-67281 Kirchheim (DE)

(56) Entgegenhaltungen:
- EP-A- 0 150 965
- EP-A- 0 377 503
- WO-A-93/02364
- WO-A-94/01780
- DE-A- 4 035 052
- FR-A- 80 331
- FR-A- 2 361 651
- FR-A- 2 476 350
- GB-A- 258 105
- US-A- 3 651 927
- US-A- 4 328 184
- US-A- 5 167 922
- PATENT ABSTRACTS OF JAPAN vol. 8, no. 129 (P-280) 15. Juni 1984 & JP-A-59 032 851 (KONISHIROKU SHASHIN KOGYO)
- PATENT ABSTRACTS OF JAPAN vol. 7, no. 172 (P-213) 29. Juli 1983 & JP-A-58 077 663 (KIYOUTO DAIICHI KAGARU KK)
- PATENT ABSTRACTS OF JAPAN vol. 9, no. 40 (P-336) 20. Februar 1985 & JP-A-59 180 461 (KONISHIKORU SHASHIN KOGYO KK)
- PATENT ABSTRACTS OF JAPAN vol. 10, no. 177 (P-470) 21. Juni 1986 & JP-A-61 026 864 (KONISHIROKU PHOTO IND CO LTD)
- PATENT ABSTRACTS OF JAPAN vol. 10, no. 67 (C-333) 15. März 1986 & JP-A-60 207 598 (KOBAYASHI SEIYAKU KK)
- PATENT ABSTRACTS OF JAPAN vol. 9, no. 182 (P-376) (1905) 27. Juli 1985 & JP-A-60 055 264 (FUJI)

## Beschreibung

Die Erfindung betrifft ein System zur Analyse von Probenflüssigkeiten, das eine mechanische Vorrichtung zur Beförderung von Testelementen, eine Meßanordnung zur Detektierung von auf einem Testelement auftretenden Veränderungen und zwei oder mehr einzeln versiegelte Testelemente innerhalb des Systems beinhaltet. Weiterhin betrifft die Erfindung ein Verfahren zur Analyse von Probenflüssigkeiten.

Systeme, die eine Analyse ermöglichen, ohne daß ein Hantieren mit flüssigen Reagenzien notwendig ist, werden in erster Linie im medizinischen Bereich und in der Umweltanalytik eingesetzt. Es handelt sich hierbei um sogenannte Trockentests. Bei diesen Systemen sind analytische Tests für einzelne Parameter möglich, die ohne Kenntnis der chemischen oder biologischen Zusammenhänge benutzt werden können und bei Einhaltung der Anleitung zu genauen Meßergebnissen führen. Solche Systeme können daher von ungeschulten Personen bedient werden, was ihnen besonders im medizinischen Bereich bei der Benutzung durch Patienten großen Stellenwert verschafft hat.

In den letzten Jahren haben Geräte zur Kontrolle des Blutzuckerspiegels durch den Patienten zunehmend an Bedeutung gewonnen. Die Verwendung von Geräten, die ständig mitgeführt werden können, verschafft dem Patienten eine weitgehende Unabhängigkeit vom behandelnden Arzt und damit einen Gewinn an Lebensqualität. Die Möglichkeit einer einfachen und schnellen Messung ermöglicht eine verbesserte Medikamentierung, z. B. die Einstellung des Blutzuckerspiegels, durch die Gabe von Insulin oder Zucker nach dem Bedarf des Organismus. Auch die Lebensweise des Patienten kann auf diese Weise den aktuellen Bedürfnissen des Körpers angepaßt werden. Eine Verwendung tragbarer und vom Patienten zu benutzender Geräte wird sich in Zukunft zunehmend auch auf weitere Blutinhaltsstoffe ausdehnen. Neben körpereigenen Stoffen ist hier an die Kontrolle von Medikamentenkonzentrationen im Blut zu denken. Besonders wichtig wird eine solche Kontrolle, wenn die pharmakologische Breite eines Therapeutikums gering ist wie z. B. bei Digitalis-Steroiden oder Lithium.

Bei bisherigen Ausführungsformen von Meßgeräten für Inhaltsstoffe von Körperflüssigkeiten sind Meßgerät und Testreagenzien voneinander getrennt. Die Testreagenzien liegen z. B. in Form von Teststreifen vor, auf welche die Körperflüssigkeit aufgegeben wird. Zur Messung wird der Teststreifen in ein Meßgerät eingeführt. Bei vielen Reagenzien wird die Lagerbeständigkeit durch Feuchtigkeit stark herabgesetzt. Eine bekannte Möglichkeit, die Lagerbeständigkeit der Testelemente über einen längeren Zeitraum zu gewährleisten, besteht darin, sie in eine Metallfolie einzusiegeln. Dies ist beispielsweise für Harnteststreifen üblich. Eine weitere Möglichkeit, feuchtigkeitsempfindliche Reagenzien zu lagern ist, eine Vielzahl von Testelementen in Gefäßen, d. h. in der Regel Röhrchen, aus für Luftfeuchtigkeit undurchlässigem Material aufzubewahren. Aus diesen Gefäßen werden die Testelemente einzeln entnommen, wobei jedoch beim Öffnen des Gefäßes Umgebungsluft und damit Feuchte eindringt. Um eine Lagerbeständigkeit der verbleibenden Testelemente zu gewährleisten, befinden sich Trockenmittel im Innern des Gefäßes, die eingetretene Feuchte aufnehmen. Der Benutzer eines Analysensystems muß daher in den beschriebenen Fällen neben einem Meßgerät außerdem getrennt einen Vorrat an Testelementen bei sich führen.

Ein im Stand der Technik bekannter Prozeß zur Analyse einer Probenflüssigkeit, beispielsweise zur Bestimmung von Glucose in Blut, besitzt die folgenden Verfahrensschritte:
- manuelle Entnahme eines Teststreifens aus einem separaten Vorratsgefäß
- Aufgabe der Probenflüssigkeit
- Einbringen des Testelementes in das Meßgerät
- Durchführung der Messung
- Ablesen des Meßwertes
- Entnahme des gebrauchten Testelementes

Bei der Entnahme des Testelementes aus einem separaten Gefäß benötigt man die schon genannten Vorkehrungen gegen Luftfeuchtigkeit. Ferner besteht die Gefahr der Kontamination des Teststreifens durch an den Händen haftenden Schmutz, was zu einer Verfälschung des Testergebnisses führen kann. Auch eine Verschmutzung des Teststreifens durch Herunterfallen ist denkbar. Die folgenden Schritte der Probenaufgabe und des Einbringens in das Meßgerät sind bei einigen Geräten im bekannten Stand der Technik miteinander vertauscht. Die Aufgabe der Probe erfolgt in diesen Fällen, während sich das Testelement bereits im Meßgerät befindet. Auf diese Weise wird beispielsweise die Aufgabe eines Blutstropfens aus einer zuvor angestochenen Fingerspitze erleichtert, da das Testelement durch das Meßgerät fixiert ist.

Bei bekannten Analysensystemen, die einzelne Testelemente verwenden, wird das Testelement manuell durch den Benutzer an den Ort der Messung gebracht. Um eine Fehlbenutzung infolge falscher Positionierung zu vermeiden, muß eine entsprechende Konstruktion des Gerätes gewählt werden. Testelemente in Form eines Streifens werden beispielsweise bis zum Anstoßen des Streifens in eine vorgesehene Führungsschiene eingeschoben.

Eine Analyse kann durch detektierbare Signale erreicht werden, deren Signalstärke abhängig von der Konzentration des zu bestimmenden Parameters in der Probe ist. Dem Fachmann sind für Analysen geeignete, detektierbare Signale, z. B. optischer, elektrischer oder magnetischer Art, bekannt.

Die Durchführung der Messung erfolgt bei Geräten, die Testelemente verwenden, in der Regel reflektrometrisch. Weniger gebräuchlich sind Geräte, die transparente Testelemente verwenden, bei denen die Detektion in Transmission erfolgt. Die Auswertung der Meßergebnisse erfolgt mit elektronischen und in der Regel auch digitalen Methoden.

Analysengeräte, die eine Vielzahl von Testelementen enthalten, sind bisher auf Labors oder größere Arztpraxen beschränkt gewesen, da hier hohe Durchsätze an einzelnen Tests erreicht werden, die zusätzliche Vorkehrungen gegen Luftfeuchte, Lichteinfluß etc. überflüssig machen. In der Patentanmeldung EP-A-0513618 wird beispielsweise eine radiale Anordnung von Testelementen beschrieben. Die Testelemente werden von einer mechanischen Vorrichtung der Anordnung entnommen und Probenflüssigkeit wird mit einer Pipette aufgegeben. Im Gegensatz zur vorliegenden Erfindung sind die Testelemente jedoch innerhalb des Analysengerätes unversiegelt. Diese Anordnung ist daher zum Verbrauch aller Testelemente innerhalb einer kurzen Zeitspanne in der Größenordnung von Minuten oder Stunden konzipiert.

Im Stand der Technik ist weiterhin das Dokument WO 93/2364 bekannt, in dem ein Laborgerät mit einem Karussell beschrieben ist. Das Karussell besitzt eine Vielzahl von Kammern, in denen sich Testelemente befinden. Zur Entnahme von Testelementen wird eine an der Frontseite der Kammern befindliche Folie mit einer Schneidevorrichtung (Cutter) geöffnet und das Testelement darauf mit einem Greifarm aus der Kammer herausgezogen.

Der Erfindung lag die Aufgabe zugrunde, ein Analysensystem zur Verfügung zu stellen, bei dem mehrere Testelemente innerhalb des Systems so vorliegen, daß eine hohe Lagerstabilität der Testelemente auch nach der Entnahme einzelner Elemente garantiert werden kann.

Demgemäß wurde ein System zur Analyse von Probenflüssigkeiten konzipiert, das eine mechanische Vorrichtung zur Beförderung von Testelementen an den Ort der Messung und eine Meßanordnung zur Detektierung von auf einem Testelement auftretenden Veränderungen besitzt und bei dem sich zwei oder mehr Testelemente im System befinden, die einzeln versiegelt sind.

Bestandteil der Erfindung ist ebenfalls ein zur Analyse von Probenflüssigkeiten verwendbares Verfahren unter Benutzung des erfindungsgemäßen Systems.

Ein erfindungsgemäßes System beinhaltet ein Meßgerät und eine Anordnung von Testelementen.

Ein bevorzugter Aspekt der vorliegenden Erfindung sind die zum System gehörenden Testelemente. Sie besitzen einen Grundkörper, der aus einem mechanisch stabilen Material, bevorzugt Plastik, gefertigt ist, jedoch auch Metalle, Gläser oder Kartone beinhalten kann. Dieser Grundkörper bildet bevorzugt einen flachen Körper, der in einer Aussparung ein Testfeld enthält. Mögliche Testfelder können aus mehreren Schichten bestehen. Neben einer Schicht, in der mit Hilfe des Analyten und Reagenzien ein detektierbares Signal erzeugt wird, sind auch Schichten bekannt, die Hilfsstoffe enthalten oder die eine Abtrennung von Zellen vornehmen. Ein möglicher Aufbau eines Testfeldes ist in der Patentanmeldung EP-A-0271854 beschrieben. In einem erfindungsgemäßen Testelement kann das Testfeld beispielsweise durch Einkleben oder Einpressen in den Grundkörper befestigt werden. Auf der einen Seite des Testfeldes, bevorzugt der Oberseite, kann die Aufgabe eines flüssigen Analyten erfolgen. Der Analyt dringt in das Testfeld ein, wobei z. B. Zellen abgetrennt werden können und/oder eine Reaktion mit unter Umständen mehreren Hilfsstoffen stattfindet. In einer der möglichen Schichten, bevorzugt derjenigen, die sich an der Unterseite des Testelementes befindet, wird eine detektierbare Veränderung in Abhängigkeit vom Analyten hervorgerufen. Vorzugsweise ist diese detektierbare Veränderung eine Farbänderung, es sind jedoch auch andere detektierbare Eigenschaften, wie z. B. Änderung der magnetischen oder elektrischen Eigenschaften oder Lichtemission, möglich. Die Formgebung der Testelemente wird zwar auch durch die für die Detektion notwendigen Schichten des Tests beeinflußt. Bevorzugt sind jedoch solche Testelemente, die aufgrund ihrer mechanischen Stabilität und/oder Form ein Herausschieben, Herausdrücken etc. aus einer Einsiegelung möglich machen. Aus diesem Grund sind ebenfalls solche Testelemente bevorzugt, die eine Spitze oder Kante, besonders bevorzugt in eine Transportrichtung, aufweisen. Bevorzugte Ausführungsformen der Testelemente besitzen ferner Führungselemente, die einen Transport an den Ort der Messung begünstigen. Außerdem besitzen bevorzugte Ausführungsformen der Testelemente einen Angriffspunkt für einen Transportmechanismus.

Die Detektion von Veränderungen auf dem Testelement kann durch dem Fachmann prinzipiell bekannte Meßanordnungen erfolgen, die eine Strahlungsquelle und einen Strahlungsdetektor beinhalten. Die zur Messung verwendete Strahlung liegt bevorzugt im sichtbaren Bereich des Frequenzspektrums. Die von der Strahlungsquelle ausgesandte Strahlung trifft für den bevorzugten Fall einer Reflexionsanordnung entweder direkt oder nach dem Durchqueren einer Optik auf die Unterseite des Testfeldes, von dem Strahlung reflektiert wird. Die reflektierte Strahlung trifft direkt oder durch eine Optik auf den Strahlungsempfänger. Der Strahlungsempfänger kann beispielsweise eine Photodiode, ein Photomultiplier oder ein photovoltaisches Element sein. Die obengenannte Optik kann sowohl optische Linsen als auch Spiegel beinhalten. Außerdem sind Beugungsgitter, Prismen und optische Filter gebräuchlich, die aus dem in der Regel breiten Frequenzbereich der Strahlungsquelle einen gewünschten, schmalen Frequenzbereich herausselektieren. Es sind außerdem Meßanordnungen möglich, bei denen die geschilderte Selektion eines schmalen Frequenzbereiches nach der Reflektion vom Testelement, jedoch vor dem Eintritt in den Strahlungsempfänger erfolgt. Die Auswertung der aufgenommenen Strahlung kann in bekannter Weise, durch eine elektronische Schaltung erfolgen und durch eine Anzeigevorrichtung dargestellt werden. Es ist mit geeigneten Strahlungsempfängern jedoch auch möglich, das Signal direkt auf einer Anzeigenvorrichtung darzustellen. Eine Anzeigevorrichtung kann z. B. ein analoges Display sein, bevorzugt wird es jedoch ein digitales Display sein.

Im Rahmen der Erfindung wird weiterhin eine mechanisch zusammenhängende Anordnung von Testelementen beschrieben, die, dadurch gekennzeichnet ist, daß jedes enthaltene Testelement einzeln versiegelt ist. Unter Einzelversiegelung wird die luft- und wasserdichte Abtrennung von einzelnen Testelementen, sowohl von der Umgebung außerhalb der Einsiegelung als auch voneinander, verstanden. Die Testelemente dieser Anordnung entsprechen den weiter oben beschriebenen, Testelementen. Eine getrennte Versiegelung jedes einzelnen Elementes erfolgt bevorzugt durch Einsiegelung von Testelementen in einen Blister. Besonders bevorzugt ist eine radiale Anordnung der versiegelten Testelemente, beispielsweise in Form einer Diskette, die daher nachfolgend Testelemente-Diskette genannt wird.

Im Rahmen der Erfindung können insbesondere einzelne Testelemente verwendet werden, die
a) eine Spitze oder Kante besitzen, die ein Durchstechen einer Einsiegelung beim Herausschieben aus der Einsiegelung erleichtert, und/oder
b) eine Einkerbung besitzen, die als Angriffspunkt für eine Mechanik dient,
   oder
c) Führungselemente besitzen, die bei Bewegung parallel zu den gegenüberliegenden Seiten in einer die Führungselemente aufnehmenden Schiene eine Bewegung senkrecht zur Hauptbewegungsrichtung Verhindern.

Mit einem erfindungsgemäßen System ist ein Verfahren zur Analyse von Probenflüssigkeiten möglich, das ebenfalls zur Erfindung gehört. Das Verfahren zur Analyse von Probenflüssigkeiten besitzt die Schritte:
- Entnahme eines Testelementes aus seiner Versiegelung durch eine Vorrichtung,
- Transport des Testelementes an den Ort zur Probenaufgabe,
- Aufgabe einer Probe auf das Testelement,
- Ablesen des Meßergebnisses.

Bei dem Verfahren wird zur Analyse von Probenflüssigkeiten ein Testelement bevorzugt aus einem Blister entnommen, wozu bevorzugt ein Dorn verwendet wird, der sich an einer Vorrichtung befindet, die ebenfalls zum Transport des Testelementes dient. Die Aufgabe der Probenflüssigkeit erfolgt auf das Reagenzfeld eines entsiegelten Testelementes. Die Messung kann mit einer Meßvorrichtung, wie sie weiter oben beschrieben wurde, erfolgen. Bevorzugt wird die Messung an einer der Probenaufgabe gegenüberliegenden Seite des Testelementes durchgeführt.

Bei einem erfindungsgemäßen System befinden sich mehrere einzeln versiegelte Testelemente innerhalb des Systems. In einer bevorzugten Ausführungsform liegen die Testelemente in einer mechanisch zusammenhängenden Form vor. Besonders eine kreisförmige Anordnung der Testelemente in Form einer Diskette ist vorteilhaft, die einen radialen Transport der Kammern ermöglicht. Außerdem ist die Anordnung platzsparend, so daß eine Vielzahl von Testelementen im Gerät untergebracht werden können. Die Herstellung einer solchen Diskette kann analog der Herstellung von Blistern für Tabletten erfolgen. Blister werden im allgemeinen aus zwei Folien gefertigt, von denen die erste Vertiefungen aufweist, in die Tabletten oder im erfindungsgemäßen Fall die Testelemente eingelegt werden. Eine zweite Folie wird mit der ersten Folie verschweißt oder verklebt. Dies führt zu einer Einsiegelung der Testelemente, bei der die Testelemente einzeln und voneinander durch Stege getrennt, eingesiegelt sind. Als Material für die Folien sind z. B. Kunststoffe möglich, erfindungsgemäß sind jedoch kunststoff-laminierte Metallfolien bevorzugt, da diese einen besseren Schutz gegen Feuchtigkeit, Licht und Verschmutzung bieten. Besonders bevorzugt sind Folien, bei denen sich eine Aluminiumschicht auf einer Polyethylenschicht befindet und die Aluminiumschicht mit einem Außenlack überzogen ist. Die Entnahme einzelner Testelemente aus der Versiegelung kann prinzipiell durch den Benutzer manuell erfolgen, wird jedoch bevorzugt durch einen Mechanismus vorgenommen. Besonders bevorzugt ist ein Mechanismus, der sowohl die Entsiegelung eines Testelementes als auch den Transport dieses Elementes an den Ort der Messung vornehmen kann.

Auf der Testelemente-Diskette können Daten codiert sein, welche den Inhalt der Diskette, das Verfalldatum der Testelemente, die Herstellungscharge und andere Daten betreffen. Die Codierung kann durch einen Strichcode, ein Lochmuster, einen Magnetstreifen oder andere, dem Fachmann bekannte Möglichkeiten, realisiert sein. Es kann ebenfalls mit der Testelemente-Diskette ein Codestreifen oder ein Code-Key, z. B. in Form eines radio-frequency identification keys (RF-ID) mitgeliefert werden, der bereits genannte Daten trägt. Ein Lesegerät für die jeweilige Datenform kann in das System integriert sein.

Die von der Testelemente-Diskette gelesenen Daten können die Handhabung des Gerätes wesentlich verbessern, indem beispielsweise gewarnt wird, wenn die Haltbarkeit der Testelemente überschritten wurde oder indem bei der Auswertung der Messung eine chargenspezifische Auswertekurve zugrundegelegt wird.

Die Figuren 1 bis 7 zeigen exemplarisch eine besonders bevorzugte Ausführungsform des Systems.
- Figur 1:: Gesamtes Meßgerät, bei dem ein innerer und ein äußerer Deckel aufgeklappt sind.
- Figur 2:: Aufsicht auf den Grundkörper des Meßgerätes ohne inneren und äußeren Deckel.
- Figur 3:: Aufsicht auf den Grundkörper des Meßgerätes ohne inneren und äußeren Deckel und ohne Träger für die Testelemente-Diskette.
- Figur 4:: Innerer Deckel in Ansicht von unten.
- Figur 5:: Testelemente-Diskette
- Figur 6:: Einzelnes Testelement
- Figur 7:: Äußerer Deckel in Ansicht von unten mit zusätzlicher Testelemente-Diskette.

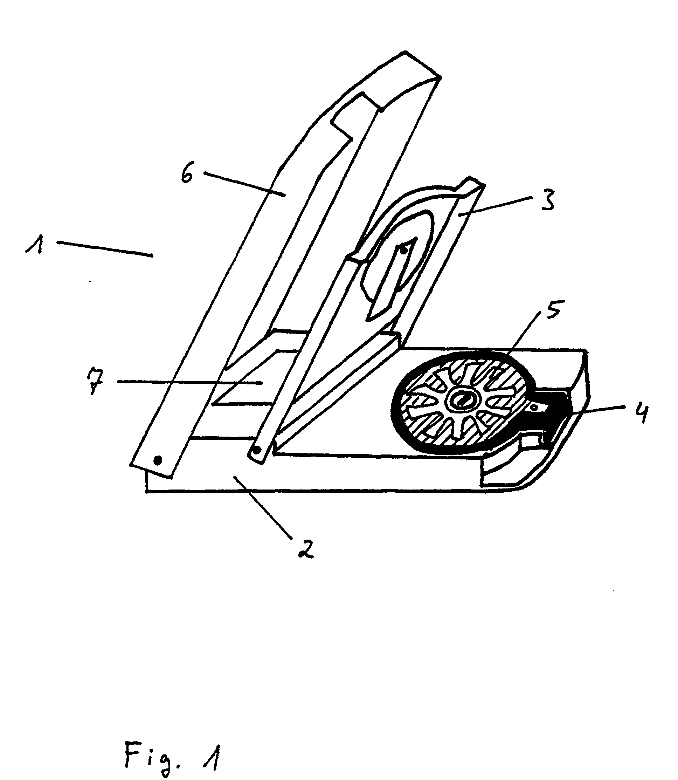

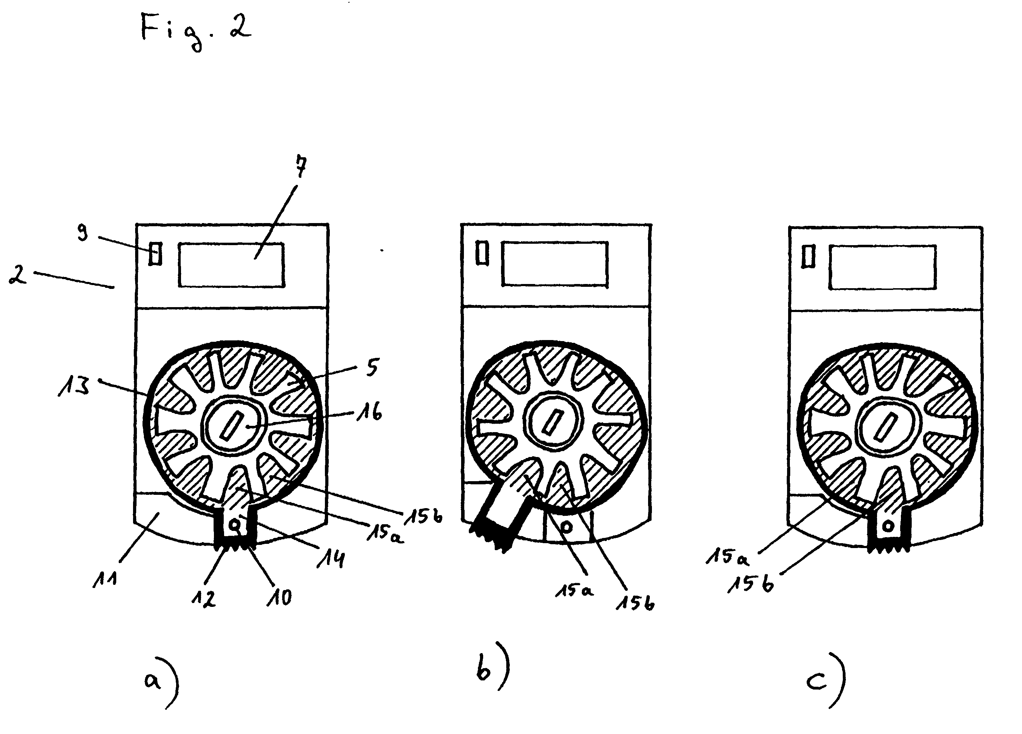

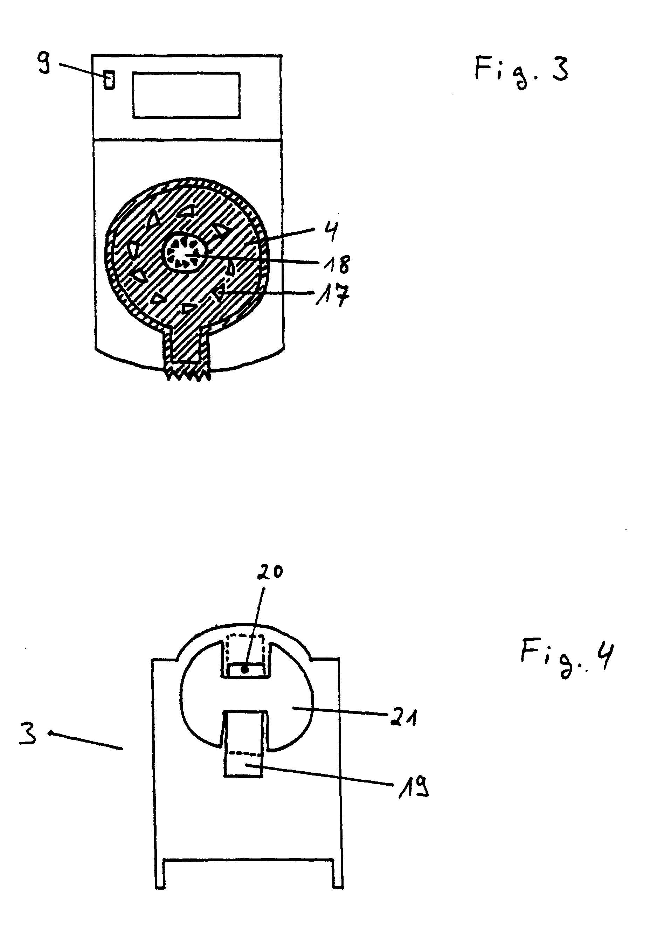

Eine Ausführungsform des Analysensystems mit Testelemente-Diskette ist in Figur 1 dargestellt. Ein erfindungsgemäßes System gehört zu den Analysensystemen, bei denen der Analyt auf ein Testelement aufgegeben und in einem dafür vorgesehenen Meßgerät ausgewertet wird. Testelement und Meßgerät sind sowohl in Form als auch bezüglich des Meßsignals aufeinander abgestimmt, so daß ihre Kombination als System zu bezeichnen ist. Der Raum, in den die Testelemente-Diskette eingelegt werden kann, wird durch den inneren Deckel (3) verschlossen. Der Grundkörper (2) enthält einen ersten Transportmechanismus, bestehend aus einem Hebel (4) und einem drehbaren Halter (5) für eine Testelemente-Diskette. Der äußere Deckel (6) dient zum Schutz des Gerätes vor mechanischen Einwirkungen und zur Arretierung des Hebels (4). Messungen erfolgen bevorzugt bei geöffnetem äußeren Deckel (6), es ist jedoch ebenfalls möglich bei geschlossenem äußeren Deckel (6) zu messen, der einen zusätzlichen Schutz gegen Umgebungslicht darstellt. Bei geschlossenem äußeren Deckel (6) ist ebenfalls eine gewünschtenfalls im Grundkörper (2) befindliche Vorrichtung zur Anzeige von Meßergebnissen (7) gegen mechanische Einwirkungen geschützt. In Figur 2 ist der Grundkörper (2) des Meßgerätes dargestellt. Auf der einen Seite des Hebels (4) befindet sich eine Vertiefung (11), so daß der Hebel radial in diese Richtung bewegt werden kann, während eine Bewegung in die entgegengesetzte Richtung aus der in Figur 2a) dargestellten Position unmöglich ist. Der Hebel (4) besteht aus einem rechteckigen vorderen Teil (12) und einer Kreisscheibe (13). Der rechteckige vordere Teil des Hebels (12) hat einen Ausschnitt (14). Die Seiten des Ausschnitts (14) sind so geformt, daß sie ein Testelement führen können, wenn dies in den Ausschnitt eingeschoben wird. Diese Führungselemente verhindern ein Herausfallen des Testelementes während der Messung, ermöglichen jedoch den Auswurf des Testelementes nach Abschluß der Messung. Während der Messung befindet sich das Testelement innerhalb des Ausschnittes (14) und die Führungselemente dienen dazu, die Positionierung für den Meßvorgang sicherzustellen. Unterhalb des Ausschnitts (14) befindet sich integriert in den Grundkörper (2) eine Meßanordnung, die aus einer Lichtquelle und einem Detektor besteht. In Figur 2 ist eine Meßöffnung (10) dargestellt, durch die sowohl von der Lichtquelle ausgesandtes Licht, als auch das vom Testelement reflektierte Licht hindurchtreten kann. Die Lichtquelle kann bereits beschriebene und im Stand der Technik bekannte mono- als auch polychromatische Ausführungsformen aufweisen. Für den Detektor sind ebenfalls bekannte Ausführungsformen möglich, wobei jedoch Detektoren für den sichtbaren und infraroten Bereich des Spektrums bevorzugt sind. Die detektierten Signale werden durch eine Elektronik innerhalb des Grundkörpers (2) auf übliche Weise verarbeitet und das Ergebnis in einer dem Benutzer verständlichen Form auf der Anzeigevorrichtung (7) dargestellt. Auf der Kreisscheibe (13) des Hebels liegt der bereits beschriebene Halter (5) für die Testelemente-Diskette auf. Der Halter (5) besitzt Aussparungen (15), in denen sich die eingesiegelten Testelemente (25) befinden, sofern eine Testelemente-Diskette (22) auf dem Halter (5) liegt. Der Kreisring (13) des Hebels, der Halter (5) für die Testelemente-Diskette und der Grundkörper (2) des Gerätes sind durch eine Schraube (16) miteinander drehbar verbunden. Wird die Schraube (16) entfernt, so kann der Halter (5) entnommen werden. Die verbleibende Anordnung des Grundkörpers (2) mit dem Hebel (4) ist in Figur 3 dargestellt. Auf dem Hebel (4) sind Rampen (17) angebracht, die ein Drehen des Halters (5) auf dem Hebel (4) in einer Richtung ermöglichen, jedoch ein Drehen in Gegenrichtung verhindern. Eine innere Scheibe (18) ist mit dem Grundkörper (2) fest verbunden. Auf dieser inneren Scheibe (18) sind ebenfalls kleine Rampen angebracht, die die gleiche Orientierung wie die auf dem Hebel (4) besitzen. Wird ausgehend von der Anordnung in Figur 2a eine Hebelbewegung im Uhrzeigersinn ausgeführt, so wird der Halter (5) um den gleichen Winkel im Uhrzeigersinn mitgeführt, den der Hebel überstreicht. In Figur 2b ist der Grundkörper des Meßgerätes nach Ausführung der maximalen Drehung dargestellt. Im gezeichneten Beispiel einer Diskette, die 10 Testelemente enthält, wird vom Hebel ein Winkel von 36° überstrichen. Im allgemeinen ergibt sich für eine Diskette mit n Testelementen ein Winkel von 360°/n Grad. Wird der Hebel im Gegenuhrzeigersinn in die in Figur 2 c dargestellte Position zurückgebracht, so behält der Halter (5) die in Figur 2b dargestellte Position bei. Dieser Mechanismus dient dazu, mit einer Bewegung des Hebels ein neues Testelement der Testelemente-Diskette vor dem Ausschnitt (14) des Hebels zu positionieren. Die in Figur 2 dargestellte Bildsequenz zeigt, wie die in Figur 2a vor dem Detektor positionierte Aussparung (15a) im Halter um 36° im Uhrzeigersinn wandert und eine neue Aussparung (15b) vor dem Detektor positioniert wird. Der Hebel (4) stellt daher in Verbindung mit dem Halter (5) einen radialen Transportmechanismus dar.

Figur 4 zeigt die dem Grundkörper (2) zugewandte Seite des inneren Deckels (3) des Gerätes. Ist dieser heruntergeklappt, so befindet er sich horizontal über dem Grundkörper (2) und somit auch über einer eingelegten Testelemente-Diskette. In dem inneren Deckel (3) befindet sich ein zweiter Transportmechanismus in Form eines Schiebers (19), der an einem Ende einen Dorn (20) trägt. Die mit durchgezogenen Linien eingezeichnete Position stellt die Ruheposition des Schiebers (19) dar. Befindet sich der Schieber in dieser Position, so ist eine Betätigung des Hebels (4) möglich. Wird der Schieber (19) in die gestrichelte Position geschoben, so wird das Ende des Schiebers, das den Dorn (20) trägt, in Richtung des Grundkörpers (2) abgesenkt. Ist eine Testelemente-Diskette eingelegt, so senkt sich der Dorn (20) auf ihre Oberfläche und durchsticht sie. Durch eine Weiterbewegung des Schiebers in Richtung der Aussparung (14), schiebt der Dorn ein Testelement in die Aussparung (14) des Hebels. Dabei durchsticht auch das Testelement die durch die Testelemente-Diskette gegebene Einsiegelung. Das Testelement befindet sich nach den beschriebenen Operationen in der Aussparung des Hebels (14) oberhalb des Detektors (10). In dieser Position kann die Probenaufgabe erfolgen.

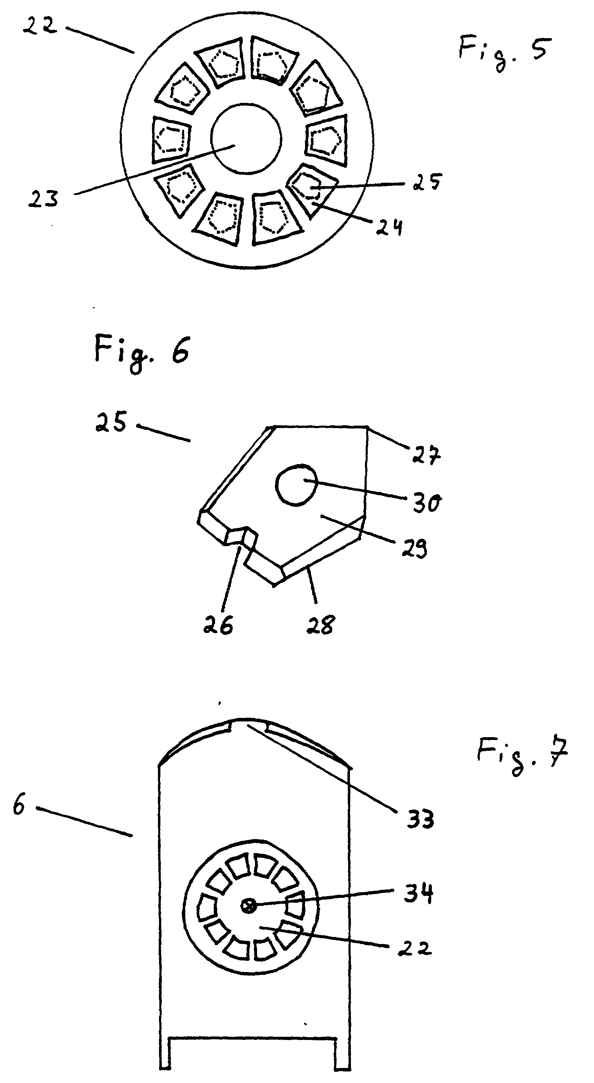

Figur 5 zeigt eine Testelemente-Diskette (22). Sie besitzt in ihrer Mitte eine kreisrunde Aussparung (23), die beim Einlegen in das Gerät locker auf der Befestigungsschraube (16) zu liegen kommt. Dies fixiert die Testelemente-Diskette (22) in der Weise, daß lediglich eine Rotation um die durch die Befestigungsschraube gegebene Achse möglich ist. Die Testelemente-Diskette kann analog den bereits beschriebenen Blistern für Tabletten hergestellt sein. Bevorzugt werden als Material für die Testelemente-Diskette kunststoff-laminierte Metalle verwendet, da diese für Feuchte undurchlässig sind, wobei Aluminium als Metall besonders bevorzugt ist. Für weniger feuchtigkeitsempfindliche Testelemente sind außerdem Kunststoffe und Kartone möglich. Die Testelemente befinden sich in Zellen (24), die durch Stege voneinander getrennt sind und gegenüber der Umgebung abgeschlossen sind, so daß die Testelemente (25) vor Feuchte, Lichteinwirkung, Verschmutzung etc. geschützt sind. Der mechanische Kontakt, der einen Weitertransport, beziehungsweise eine Drehung der Testelemente-Diskette ermöglicht, wird durch die Zellen (24) in den Testelemente-Disketten hergestellt. Die Zellen (24) bilden mit den enthaltenen Testelementen (25) Erhebungen gegenüber der übrigen Testelemente-Diskette (22). Diese Erhebungen befinden sich bei in das Gerät eingelegter Testelemente-Diskette (22) in den dafür vorgesehenen Aussparungen (15) im Halter (5).

Die Figur 6 zeigt ein einzelnes Testelement (25). Der Aufbau eines Testelementes aus Grundkörper (29) und Testfeld (30) wurde bereits beschrieben. In einer bevorzugten Ausführungsform ist der Grundkörper (29) aus einem Kunststoff gefertigt und das Testfeld (30) wird auch als Meßfeld verwendet. Das Meßfeld an der Unterseite des Testelementes (25) befindet sich bei geeigneter Positionierung oberhalb der Meßöffnung (10). Die bevorzugte Form des Testelementes besitzt eine Spitze (27), die ein Durchstechen der Abdeckfolie der Testelemente-Diskette beim Herausschieben erleichtert. Am anderen.Ende trägt das Testelement eine Einbuchtung (26), in die sich der Dorn (20) beim Herausdrücken des Testelementes (25) schiebt. Dies gewährleistet eine sichere Führung des Testelements und verhindert ein Abrutschen des Dorns. Die seitlichen Kanten (28) des Testelements sind so beschaffen, daß sie sich in die seitlichen Führungselemente der Aussparung (14) des Hebels einpassen. Auf diese Weise kann eine sichere Führung des Testelementes beim Einschieben in die Meßposition gewährleistet werden. Die seitlichen Kanten (28) des Testelementes (25) können zum Beispiel als herausstehende Kanten ausgeführt sein. Ist das Testelement an den Meßplatz geschoben, so ist ein Drehen des Hebels (4) unmöglich, da das Testelement durch den Dorn arretiert ist.

Eine Arretierung des Hebels (4) wird außerdem durch die äußere Klappe (6) erreicht, die in Figur 7 dargestellt ist. Ein Spalt (33) im vorderen Teil des Deckels umfaßt im geschlossenen Zustand den vorspringenden Teil des Hebels (4). Der äußere Deckel (6) kann an seiner Innenseite eine Halterung (34) für eine weitere Testelemente-Diskette (22) besitzen.

In dem Grundkörper des Gerätes kann außerdem eine Schaltervorrichtung (9) integriert sein, die beim öffnen oder Schließen des äußeren Deckels (6) betätigt wird. Mit dessen Signal kann das Meßgerät an- und abgeschaltet werden. Mit dieser bevorzugten Ausführungsform spart der Benutzer die Handhabungsschritte des An- und Abschaltens ein.

Die Durchführung einer Messung mit dem beschriebenen Gerät erfolgt mit den Schritten:
1. Öffnen der äußeren Klappe (6) (Meßgerät schaltet sich ein).
2. Öffnen der inneren Klappe (3).
3. Einlegen einer Testelemente-Diskette auf den Halter (5).
4. Schließen der inneren Klappe (3).
5. Freisetzen eines Testelementes durch Bewegung des Schiebers (19) von der Ruheposition (durchgezogene Linien in Figur 4) in die gestrichelt eingezeichnete Position. Das Testelement wird dadurch aus der Testelemente-Diskette in die Aussparung (14) im Hebel (4) gedrückt.
6. Probenaufgabe auf das Testelement (Messung wird gestartet).
7. Ablesen des Meßwertes auf der Anzeigevorrichtung (7) nach Abschluß der Messung.
8. Zurückbewegen des Schiebers (19) in die Ruheposition.
9. Bewegung des Hebels von der in Figur 2a dargestellten Position in die in Figur 2b dargestellte Position. Das Testelement fällt nach dieser Bewegung in die Vertiefung (11).
10. Zurückbewegen des Hebels in die in Figur 2c dargestellte Position.
11. Herauskippen des gebrauchten Testelementes aus dem Gerät.
12. Schließen der äußeren Klappe (6), sofern nicht eine erneute Analyse vorgenommen werden soll.

Befindet sich bereits eine Testelemente-Diskette im Gerät, so umfaßt der Meßprozeß lediglich die Schritte 5. bis 11.

Zum Entfernen einer leeren Testelemente-Diskette werden die beiden Klappen (6) und (3) geöffnet und die Diskette wird entnommen.

Die Schritte 8 und 10 können bei der Handhabung eingespart werden, wenn der Schieber (19) und der Hebel (4) durch Federn zurückbewegt werden.

Die beschriebene Konstruktion stellt exemplarisch die Realisierung eines erfindungsgemäßen Gerätes dar. Charakteristisch für ein solches System ist das Vorhandensein mehrerer einzeln eingesiegelter Testelemente innerhalb des Systems und die Beförderung einzelner Testelemente aus der Einsiegelung an den Ort der Messung. Ein erfindungsgemäßes System bietet daher dem Anwender den Vorteil, einige Tests hintereinander durchführen zu können, ohne einem separaten Gefäß mit der Hand ein neues Testelement entnehmen zu müssen. Im vorliegenden Fall kann die Bestimmung mit nur einem Gerät durchgeführt werden, ohne daß ein weiteres Behältnis zur Aufbewahrung der Testelemente notwendig ist. Aufgrund der Einzeleinsiegelung ist das Gerät für Testelemente, die gegen eine Komponente der Umgebung (z. B. Feuchtigkeit, Luftsauerstoff, Licht) empfindlich sind, besonders geeignet, da die Testelemente durch die Einsiegelung geschützt werden können. Die beschriebene Durchführung einer Messung vermeidet Fehlbenutzungen durch Positionierung der Testelemente, da durch den beschriebenen Mechanismus die Testelemente direkt an den Ort der Messung gebracht werden. Der Auswurfmechanismus ermöglicht weiterhin eine Entsorgung benutzter Elemente ohne eine Kontaminierung des Anwenders.

### Bezugszeichenliste

- (1): Gesamtes Gerät
- (2): Grundkörper des Gerätes
- (3): Innerer Deckel
- (4): Hebel (rechteckiger Teil und Kreisscheibe)
- (5): Halter für die Testelemente-Diskette
- (6): Äußerer Deckel
- (7): Anzeigevorrichtung
- (9): Schalter
- (10): Meßöffnung
- (11): Vertiefung im Grundkörper
- (12): Rechteckiger Teil des Hebels (4)
- (13): Kreisscheibe des Hebels (4)
- (14): Aussparung im Hebel (4)
- (15): Aussparung im Halter (5) für Testelemente-Diskette
- (16): Schraube
- (17): Rampe
- (18): Innere Scheibe
- (19): Schieber
- (20): Dorn
- (21): Befestigung für Schieber an der inneren Klappe
- (22): Testelemente-Diskette
- (23): Kreisrunde Aussparung in der Testelemente-Diskette (22)
- (24): Zelle der Testelemente-Diskette (22)
- (25): Testelement
- (26): Einbuchtung des Testelements (25)
- (27): Spitze des Testelements (25)
- (28): seitliche Kanten des Testelements (25)
- (29): Grundkörper des Testelementes (25)
- (30): Testfeld
- (33): Spalt im äußeren Deckel (6)
- (34): Halterung im äußeren Deckel (6)

## Patentansprüche

1. System zur Analyse von Probeflüssigkeiten mit
- einer Testelemente-Diskette (22), in der zwei oder mehr Testelemente (25) kreisförmig angeordnet und einzeln versiegelt sind,
- einem Schieber (19), mit dem ein Testelement aus der Testelemente-Diskette herausgeschoben wird, wobei das Testelement eine Versiegelung durchstößt und an einen Ort zur Messung transportiert wird, sowie
- eine Meßanordnung zur Detektierung von auf dem Testelement auftretenden Veränderungen.

2. System gemäß Anspruch 1, bei dem die Meßanordnung eine Strahlungsquelle und einen Detektor beinhaltet.

3. System nach Anspruch 1, **dadurch gekennzeichnet, daß** es einen Transportmechanismus mit einem Hebel (4) und einem drehbaren Halter (5) beinhaltet, mit dem die Anordnung der Testelemente radial bewegt wird.

4. System nach Anspruch 1, **dadurch gekennzeichnet, daß** der Schieber (19) an einem Ende einen Dorn (20) trägt, durch den die Testelemente-Diskette angestochen und ein Testelement mit dem Dorn aus der Testelemente-Diskette geschoben wird.

5. System nach einem der vorangehenden Ansprüche, das eine Lesevorrichtung für Daten der Testelemente-Diskette aufweist.

6. System gemäß Anspruch 1, bei dem die Testelemente-Diskette aus zwei Folien gefertigt ist, die verklebt oder verschweißt sind und zwischen denen die Testelemente eingesiegelt sind.

7. System gemäß Anspruch 6, bei dem eine der Folien Vertiefungen zum Einlegen der Testelemente besitzt.

8. System gemäß Anspruch 1 oder 6, bei dem die Testelemente eine Spitze oder Kante besitzen, die ein Durchstechen der Versiegelung erleichtert.

9. Verfahren zur Analyse von Probenflüssigkeiten, mit den Schritten:
- Entsiegelung eines Testelementes (25) aus einer Testelemente-Diskette (22), in der zwei oder mehr Testelemente kreisförmig angeordnet und einzeln versiegelt sind, durch eine Vorrichtung, die einen Schieber (19) beinhaltet,
- Transport des Testelementes (25) mit dem Schieber (19) aus der Testelemente-Diskette heraus, wobei das Testelement eine Versiegelung durchstößt und an den Ort zur Probenaufgabe gelangt,
- Aufgabe einer Probe auf das Testelement (25),
- Durchführung einer Messung mit dem Testelement,
- Ablesen eines Meßergebnisses auf einen Display (7).

10. Verfahren gemäß Anspruch 9 zur Analyse von Inhaltsstoffen von Körperflüssigkeiten.

11. Verfahren nach Anspruch 9, bei dem vom System Daten eingelesen werden, welche die zur Analyse verwendeten Testelemente (25) betreffen.

12. Verfahren nach Anspruch 9, bei dem der Meßvorgang durch die Aufgabe der Probe auf das Testelement (25) in Gang gesetzt wird.

13. Verfahren gemäß Anspruch 9, bei dem sich die Testelemente-Diskette in einem Halter (5) befindet, mit dem die Testelemente-Diskette gedreht wird.

14. Verfahren gemäß Anspruch 13, bei dem die Testelemente-Diskette um einen Winkel von 360 °/n gedreht wird, wobei n die Zahl der in der Testelemente-Diskette angeordneten Testelemente ist.

15. Verfahren gemäß Anspruch 9, bei dem das Entsiegeln eines Testelementes durch Durchstechen der Testelemente-Diskette mit einem Dorn (20), der an dem Schieber (19) angebracht ist und Herausdrücken des Testelementes aus der Testelemente-Diskette erfolgt, wobei das Testelement eine Versiegelung durchstößt.

## Claims

1. System for analysing sample fluids comprising
- a test element diskette (22) in which two or more test elements (25) are placed in a circular arrangement and individually sealed,
- a slide (19) which is used to push a test element out of the test element diskette during which the test element pierces a seal and is transported to a site for measurement, and
- a measuring arrangement for detecting changes occurring on the test element.

2. System as claimed in claim 1, in which the measuring arrangement comprises a source of radiation and a detector.

3. System as claimed in claim 1, wherein it comprises a transport mechanism having a lever (4) and a rotatable holder (5) which is used to radially move the arrangement of test elements.

4. System as claimed in claim 1, wherein the slide (19) carries a spike (20) at one end which pierces the test element diskette and a test element is pushed out of the test element diskette by the spike.

5. System as claimed in one of the previous claims which has a reading device for data for the test element diskette.

6. System as claimed in claim 1, in which the test element diskette is manufactured from two foils which are glued or welded and between which the test elements are sealed in.

7. System as claimed in claim 6, in which one of the foils has depressions to insert the test elements.

8. System as claimed in claim 1 or 6, in which the test elements have a tip or edge which facilitates the piercing of the seal.

9. Method for analysing sample fluids comprising the steps:
- unsealing a test element (25) from a test element diskette (22) in which two or more test elements are placed in a circular arrangement and are individually sealed by a device which comprises a slide (19),
- transporting the test element (25) out of the test element diskette using the slide (19) during which the test element pierces a seal and reaches the site for sample application,
- applying a sample on the test element (25),
- carrying out a measurement with the test element,
- reading a measuring result on a display (7).

10. Method as claimed in claim 9 for analysing components of body fluids.

11. Method as claimed in claim 9, in which data are read by the system which relate to the test elements (25) used for the analysis.

12. Method as claimed in claim 9, in which the measurement process is started by applying the sample to the test element (25).

13. Method as claimed in claim 9, in which the test element diskette is located in a holder (5) which is used to rotate the test element diskette.

14. Method as claimed in claim 13, in which the test element diskette is rotated by an angle of 360°/n in which n is the number of test elements arranged in the test element diskette.

15. Method as claimed in claim 9, in which a test element is unsealed by piercing the test element diskette with a spike (20) which is mounted on the slide (19) and pressing the test element out of the test element diskette during which the test element pierces a seal.

## Revendications

1. Système pour analyser des liquides d'échantillon présentant :
- une disquette d'éléments de test (22), dans laquelle deux ou plusieurs éléments de test (25) sont disposés en forme de cercle et scellés individuellement,
- un curseur (19) avec lequel un élément de test est sorti par glissement de la disquette d'éléments de test, l'élément de test perçant un scellement et étant transporté à l'endroit de la mesure,
- ainsi qu'un dispositif de mesure pour la détection des modifications survenant sur l'élément de test.

2. Système selon la revendication 1, dans lequel le dispositif de mesure comporte une source de rayonnement et un détecteur.

3. Système selon la revendication 1, **caractérisé en ce qu'**il comporte un mécanisme de transport présentant un levier (4) et un dispositif de fixation (5) rotatif, avec lequel l'élément de test est déplacé dans le sens radial.

4. Système selon la revendication 1, **caractérisé en ce que** le curseur (19) porte en une extrémité un poinçon (20), par lequel la disquette d'éléments de test est perçée et **en ce qu'**un élément de test est sorti par glissement par le poinçon de la disquette d'éléments de test.

5. Système selon l'une quelconque des revendications précédentes, qui présente un dispositif de lecture des données de la disquette d'éléments de test.

6. Système selon la revendication 1, dans lequel la disquette d'éléments de test est réalisée à partir de deux films, qui sont collés ou soudés et entre lesquels les éléments de test sont scellés.

7. Système selon la revendication 6, dans lequel l'un des films présente des creux pour placer les éléments de test.

8. Système selon la revendication 1 ou 6, dans lequel les éléments de test présentent une pointe ou un bord qui facilite le percement du scellement.

9. Procédé pour l'analyse de liquides d'échantillons, présentant les étapes:
- descellement d'un élément de test (25) d'une disquette d'éléments de test (22) dans laquelle deux ou plusieurs éléments de test sont disposés en forme de cercle et scellés individuellement, par un dispositif qui comporte un curseur (19),
- transport de l'élément de test (25) avec le curseur (19) hors de la disquette d'éléments de test, l'élément de test perçant un scellement et arrivant à l'endroit de placement de l'échantillon,
- placement d'un échantillon sur l'élément de test (25),
- réalisation d'une mesure avec l'élément de test,
- lecture d'un résultat de mesure sur un affichage (7).

10. Procédé selon la revendication 9 pour l'analyse des constituants de liquides corporels.

11. Procédé selon la revendication 9, dans lequel le système lit des données qui concernent les éléments de test (25) utilisés pour l'analyse.

12. Procédé selon la revendication 9, dans lequel le processus de mesure est démarré par le placement de l'échantillon sur l'élément de test (25).

13. Procédé selon la revendication 9, dans lequel la disquette d'éléments de test se trouve dans un dispositif de fixation (5) avec lequel la disquette d'éléments de test est tournée.

14. Procédé selon la revendication 13, dans lequel la disquette d'éléments de test est tournée d'un angle de 360°/n, n étant le nombre d'éléments de test disposés dans la disquette d'éléments de test.

15. Procédé selon la revendication 9, dans lequel le descellement d'un élément de test est réalisé par le percement de la disquette d'éléments de tests par un poinçon (20), qui est disposé sur le curseur (19) et par l'extraction par pression de l'élément de test de la disquette d'éléments de test, l'élément de test perçant un scellement.
